# EUROPEAN PATENT APPLICATION

(11) **EP 3 695 773 A1**
(43) Date of publication of application: **19.08.2020**
(21) Application number: 20155750.1
(22) Date of filing: 05.02.2020
(51) Int. Cl.: A61B 3/00, A61B 3/10, A61B 3/15

(54) **OPHTHALMIC DEVICE**

(30) Priority: 18.02.2019 JP 2019026723
(71) Applicant: Tomey Corporation, Nagoya-shi, Aichi 451-0051 (JP)
(72) Inventor: BIAN, Guangchun, Aichi-ken, 451-0051 (JP); TAKATA, Hideo, Aichi-ken, 451-0051 (JP); CHEN, Shaolang, Aichi-ken, 451-0051 (JP)
(74) Representative: BSB Patentanwälte Schütte & Engelen Partnerschaft mbB

(57) **Abstract**

Configured is an ophthalmic device including: a photographing optical system that illuminates and photographs an eye to be examined, a reference movement control unit that automatically moves the photographing optical system to a reference position for photographing a reference region in the eye to be examined; and a displacement movement control unit that automatically moves the photographing optical system to a displacement position different from the reference position.

## Description

### BACKGROUND OF THE INVENTION

### (1) Field of the Invention

The present invention relates to an ophthalmic device.

### (2) Description of Related Art

Conventionally, an ophthalmic device that measures various regions in an eye to be examined by illuminating the eye to be examined and detecting the reflected light to photograph the eye to be examined has been known. For example, JP 5636405 B and JP 5645893 B each disclose a device that measures an eye to be examined by illuminating the eye to be examined with a ring-shaped light source.

### SUMMARY OF THE INVENTION

When photographing the eye to be examined, if an attempt is made to photograph the entire eye to be examined with high resolution, an optical system and an image sensor for photographing purposes become large. On the other hand, the target to be measured in the eye to be examined is, for example, a tear meniscus or the like, and is often a specific region in the eye to be examined. Therefore, a configuration including an optical system and an image sensor for photographing the entire eye to be examined with high resolution requires an excessive cost.

The present invention has been made in view of the above-described problems, and an object of the present invention is to enable measurement of each region in the eye to be examined with a simple configuration.

To achieve the above object, an ophthalmic device includes: a photographing optical system that illuminates and photographs an eye to be examined, a reference movement control unit that automatically moves the photographing optical system to a reference position for photographing a reference region in the eye to be examined; and a displacement movement control unit that automatically moves the photographing optical system to a displacement position different from the reference position.

That is, in the ophthalmic device, after automatically performing alignment for moving the photographing optical system to the reference position for photographing the reference region in the eye to be examined, the photographing optical system is moved to the displacement position different from the reference position. Therefore, the target to be measured at a position where it has been displaced from the reference region can be photographed by the photographing optical system. The target to be measured can be photographed by the photographing optical system that can photograph the target to be measured but cannot photograph the entire eye to be examined (cannot photograph the entire eye to be examined in a state of being included in the visual field). Therefore, it is possible to execute measurement of each region in the eye to be examined with a simple configuration.

### BRIEF DESCRIPTION OF THE DRAWINGS

FIG. 1 is a diagram showing an optical system used at the time of measuring intraocular pressure;
FIG. 2 is a diagram showing an optical system used at the time of measuring the eye refractive power, and an optical system used at the time of taking an image of a tear meniscus;
FIG. 3 is a block diagram illustrating an overall configuration of an ophthalmic device according to an Example of the present invention including control units;
FIG. 4A is a flowchart of movement control processing, and FIG. 4B is a flowchart of measurement processing; and
FIG. 5 is a diagram showing a display example of a taken image.

### DETAILED DESCRIPTION OF PREFERRED EMBODIMENTS

Embodiments of the present invention will be described in the following order.
(1) Configuration of ophthalmic device:
(2) Movement control processing:
(3) Measurement processing:
(4) Other Embodiments:

### (1) Configuration of ophthalmic device:

An ophthalmic device 1 according to an embodiment of the present invention includes a casing, and optical systems and control units that are used for a plurality of types of observations and measurements are provided in the casing. In this embodiment, the ophthalmic device 1 has a function of displaying an image of a tear meniscus of an eye to be examined on a display unit to observe the width of the tear meniscus, a function of measuring eye refractive power, and a function of measuring intraocular pressure. In this embodiment, an optical system for observing the width of the tear meniscus, an optical system for measuring the eye refractive power, and an optical system for measuring the intraocular pressure share part of components.

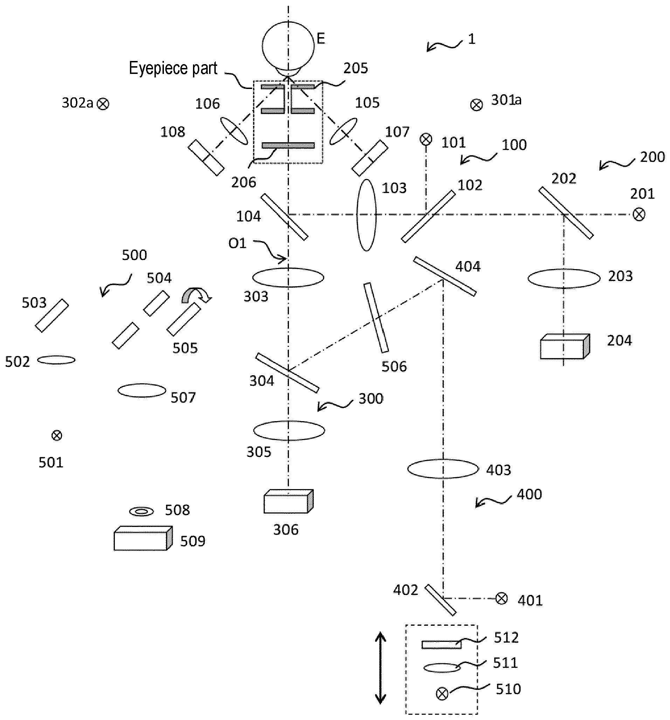

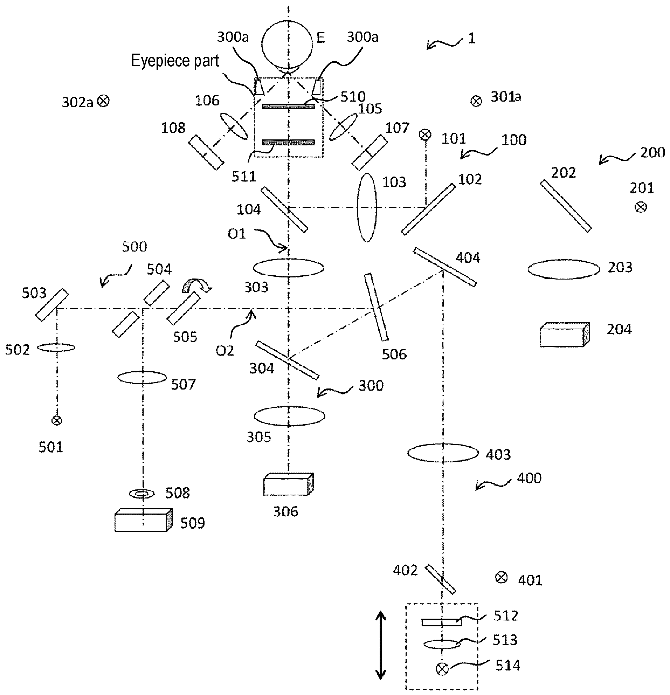

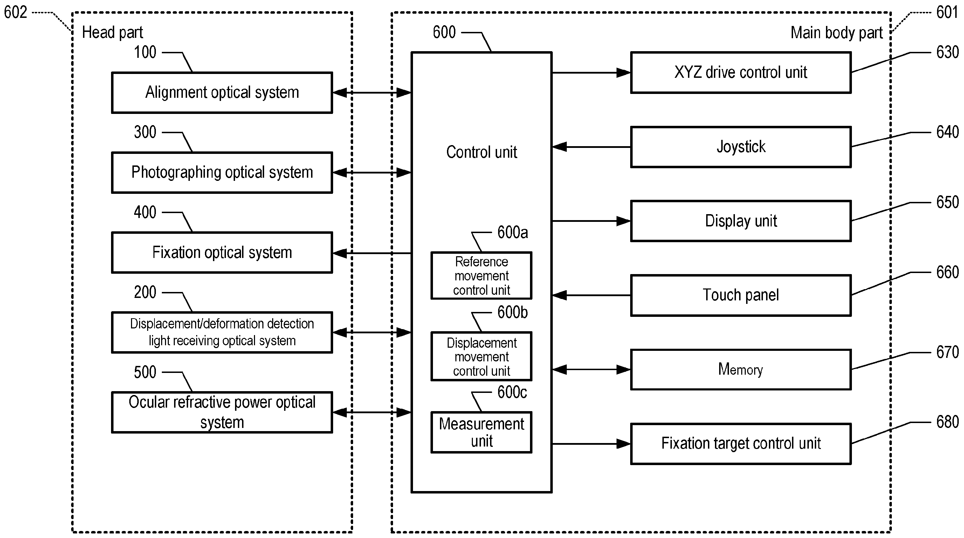

FIGS. 1 and 2 are diagrams showing these optical systems. FIG. 3 is a block diagram illustrating the overall configuration of the ophthalmic device 1 according to an Example of the present invention including control units. The ophthalmic device 1 according to an Example of the present invention will be described below with reference to FIGS. 1 to 3. As shown in FIG. 3, the ophthalmic device 1 is composed of: a head part 602 in which an optical system for measuring the eye to be examined is arranged; and a main body part 601 which includes a control unit 600 that controls, for example, the rotation motion of the optical systems and a switching unit in the head part 602.

Note that the main body part 601 is provided with: an XYZ drive control unit 630 that moves the head part 602 in XYZ (left and right, up and down, front and rear) directions relative to the main body part 601; a joystick 640 that instructs adjustment of the spatial position of the head part 602; a display unit 650 that displays an image of the photographed eye to be examined, and measurement results of the eye refractive power, the intraocular pressure and the like; a touch panel 660 that accepts instructions for measurement items and the like; a memory 670 that is used in the control processing of the control unit 600; and a fixation target control unit 680 that controls a fixation target unit (which will be described later).

### Intraocular pressure measurement optical system

FIG. 1 shows an overall optical system (intraocular pressure examination optical system) that measures the intraocular pressure of an eye to be examined. The intraocular pressure measurement optical system is provided with an alignment optical system 100 including optical elements on optical paths from a light source 101 to profile sensors 107 and 108. The intraocular pressure measurement optical system is provided with a photographing optical system 300 including optical elements on optical paths from light sources 301a and 302a to a two-dimensional image sensor (CCD) 306. Further, the intraocular pressure measurement optical system is provided with: a fixation optical system 400 including optical elements or the like on an optical path from a light source 401 through a reflection mirror 404 to an eye E to be examined; and a displacement/deformation detection light receiving optical system 200 that includes optical elements or the like on an optical path from a light source 201 to a nozzle 205 and detects the degree of deformation of the cornea of the eye to be examined. As shown in FIG. 1, a part of of the respective optical systems constituting the intraocular pressure measurement optical system are configured to be shared. In this embodiment, it is possible to switch an eyepiece part arranged in front of the eye to be examined. When the intraocular pressure is measured, the switching unit is rotated to be positioned, and a nozzle 205 for measuring the intraocular pressure is arranged in front of the eye to be examined.

### Alignment optical system 100

In the alignment optical system 100, light from the light source 101 is reflected by a hot mirror 102, passes through an objective lens 103, is reflected by a hot mirror 104, then passes through a flat glass 206 and an opening of the nozzle 205, and applied to the cornea of the eye E to be examined. The light source 101 employed in this Example is an LED that outputs infrared light.

The light reflected by (the apex of) the cornea is received by a lens 105 and the profile sensor 107 as a first detection unit and a lens 106 and the profile sensor 108 as a second detection unit, which detection units are arranged symmetrically with respect to a main optical axis O1. In this embodiment, when the position of the eye to be examined in the three-dimensional direction is an appropriate position, the position where the light reflected by the cornea is detected by the profile sensor 107 and the profile sensor 108 is determined in advance. The control unit 600 of the main body part 601 instructs the XYZ drive control unit 630 to move the head unit 602 in the three-dimensional direction so that the light reflected by the cornea is detected by the profile sensor 107 and the profile sensor 108 and detected positions of the light the position determined in advance. As a result, the head part 602 and its internal optical system are automatically aligned in the three-dimensional direction with respect to the eye to be examined. In this embodiment, (the apex of) the cornea serves as a reference region, and the reference region can be photographed by the photographing optical system 300 in a state where such automatic alignment is performed. Therefore, the aligned state is a state in which the photographing optical system 300 exists at the reference position.

Note that various techniques may be employed to perform alignment. For example, a configuration in which the control unit 600 performs auto-alignment (fine adjustment) after the examiner performs rough alignment can be employed. For the rough alignment, there can be employed, for example, a technique in which the examiner visually recognizes a bright spot due to the reflection from the cornea on the image of the eye to be examined displayed on the display unit 650, and further the joystick 640 is used to move the head part 602 so that the bright spot is positioned within a predetermined range. Of course, the alignment optical system is not limited to the examples as shown in FIGS. 1 and 2, and may be configured, for example, so that the optical system that performs alignment in the Z direction and the optical system that performs alignments in the XY directions are different optical systems which may partially overlap.

### Photographing optical system 300

The photographing optical system 300 is provided with light sources 301a and 302a. In this embodiment, the light sources 301a and 302a are light sources that output infrared rays. When the photographing optical system 300 is used at the time of measuring the intraocular pressure, an anterior ocular region including the cornea part of the eye to be examined is irradiated by the light source 301a and the light source 302a arranged on the eye to be examined side of the head part 602. In this state, the anterior ocular image of the eye to be examined is acquired by an objective lens 303, an imaging lens 305, and the two-dimensional image sensor 306, and the acquired anterior ocular image of the eye to be examined is displayed on the display unit 650. LEDs that output infrared light are employed as the light sources 301a and 302a and the light source 101 for alignment. Light having a wavelength shorter than that of the light source 101 is employed as the light sources 301a and 302a. Therefore, the hot mirror 104 transmits light for observation (observation light) and reflects light for alignment (alignment light i.e. light from the light source 101). A dichroic mirror 304 has a reflection/transmission wavelength region set so that the observation light is transmitted. Thus, the alignment light and the observation light are appropriately divided to enable each measurement.

### Fixation optical system 400

When the fixation optical system 400 is used, light (fixation light) from the light source 401 is reflected by a hot mirror 402, passes through a relay lens 403, and is reflected by a reflection mirror 404. Thereafter, the light is transmitted through a hot mirror 506, is reflected by the dichroic mirror 304, passes through the main optical axis O1, passes through the objective lens 303 and the hot mirror 104, and forms an image on the retina of the eye to be examined. Therefore, it is desirable that the light source 401 and the position of the retina of the eye to be examined be substantially conjugate. The eye to be examined is fixated based on fixation light, and eye characteristics, such as intraocular pressure measurement, can be measured. As the light source 401, an LED that outputs visible light which can be visually recognized by an subject is employed.

### Displacement/deformation detection light receiving optical system 200

When the displacement/deformation detection light receiving optical system 200 is used, part of the light from the light source 201 (deformation detection light) is transmitted through a half mirror 202, then transmitted through the hot mirror 102 and the objective lens 103, reflected by the hot mirror 104, and passes through the main optical axis O1. Further, the light passes through the flat glass 206 and the opening of the nozzle 205 and is applied to the cornea of the eye to be examined. The light applied to the cornea is reflected by the cornea, and, in the reverse path, passes through the opening of the nozzle 205 and the flat glass 206, is reflected by the hot mirror 104, and passes through the objective lens 103 and the hot mirror 102, and part of the light is reflected by the half mirror 202. Thereafter, the light is received by a light receiving element 204 by a condenser lens 203. At the time of measuring the intraocular pressure, compressed air is blown toward the cornea of the eye to be examined from an air blow port provided at the tip of the nozzle 205. When air is blown, the cornea is displaced/deformed, so that the amount of light received by the light receiving element 204 changes. The intraocular pressure value of the eye to be examined is calculated from the degree of change in amount of light. An LED that outputs infrared light is also employed as the light source 201, but light having a wavelength longer than that of the observation light and a wavelength shorter than that of the alignment light is selected and employed. In this way, the wavelengths of the alignment light, the observation light, the fixation light, and the deformation detection light (light from the light source 201) are set, and the reflection/transmission characteristics of the hot mirrors 102, 104, 506, 402 and the dichroic mirror 304 are appropriately set, so that these four lights travel along appropriate optical paths.

### Eye refractive power measurement optical system

FIG. 2 shows an overall optical system (eye refractive power measurement optical system) that measures the eye refractive power of the eye to be examined. The eye refractive power measurement optical system is provided with the alignment optical system 100 including optical elements on optical paths from the light source 101 to the profile sensors 107 and 108. The eye refractive power measurement optical system is provided with a photographing optical system 300 including optical elements on optical paths from the light sources 301a and 302a or a light source 300a to a two-dimensional image sensor 306. Further, the eye refractive power measurement optical system is provided with the fixation optical system 400 including optical elements or the like on an optical path from a light source 514 through a fixation target 512 and the reflection mirror 404 to an eye E to be examined. Further, the eye refractive power measurement optical system is composed of an eye refractive power optical system 500 that includes optical elements or the like on an optical path from a light source 501 through a mirror 503 and a flat glass 511 to the eye E to be examined, and detects the eye refractive power of the eye to be examined. As shown in FIG. 2, a part of the respective optical systems constituting the eye refractive power measurement optical system are configured to be shared. When measuring the eye refractive power, a switching unit is rotated to be positioned, and flat glasses 510 and 511 for measuring the eye refractive power are arranged in front of the eye to be examined.

Since the alignment optical system 100 and the photographing optical system 300 at the time of photographing using the light sources 301a and 302a are the same as those at the time of the above-described intraocular pressure measurement, description thereof is omitted here. The photographing optical system 300 and the fixation optical system 400 at the time of photographing using the light source 300a are partially different from those at the time of measuring the intraocular pressure, and will be described below.

### Fixation optical system 400: eye refractive power measurement

When measuring the eye refractive power, the light source 401 used at the time of measuring the intraocular pressure is turned off, and the light source 514, which is another light source, is turned on. The light from the light source 514 is collimated by a collimator lens 513 and applied to a fixation target 512. The light from the fixation target 512 is transmitted through the hot mirror 402 and the relay lens 403, then reflected by the reflection mirror 404, transmitted through the hot mirror 506, reflected by the dichroic mirror 304, and passes through the main optical axis O1. Thereafter, the light is transmitted through the objective lens 303, the hot mirror 104, and the flat glasses 511 and 510, and forms an image on the retina of the eye E to be examined. Therefore, it is desirable that the fixation target 512 and the position of the retina of the eye to be examined be substantially conjugate. The eye to be examined is fixated based on the fixation target 512. When measuring the eye refractive power, the control unit 600 outputs a control instruction to the fixation target control unit 680. As a result, the fixation target control unit 680 controls the movement of the fixation target unit (the fixation target 512, the collimator lens 513, and the light source 514) so that the fixation target and the position of the retina of the eye to be examined are substantially conjugate to fixate the eye to be examined. Thereafter, the control unit 600 outputs a control instruction to the fixation target control unit 680, and the fixation target control unit 680 moves the fixation target unit by a predetermined distance so as to establish a cloudy(foggy) state, and then measures the eye refractive power. Therefore, the fixation target unit can be moved frontward and rearward along the optical axis by a signal from the control unit 600. As the light source 514, an LED that outputs visible light which has a wavelength shorter than that of the light source 401 and can be visually recognized by the subject is employed.

### Eye refractive power optical system 500

When the eye refractive power optical system 500 is used, the light from the light source 501 (reflected light) is collected by a condenser lens 502, reflected by the mirror 503, and passes through a hole located at the center of a perforated mirror 504. Then, the light is transmitted through a parallel flat glass 505 that is arranged obliquely with respect to an optical axis O2 and rotates about the optical axis O2 by a driving unit (not shown), and then reflected by the hot mirror 506 and the dichroic mirror 304, and passes through the main optical axis O1. Then, the light is transmitted through the objective lens 303, the hot mirror 104, and the flat glasses 511 and 510 and is applied to the retina of the eye E to be examined. The reflected light from the retina of the eye E to be examined is transmitted through the flat glasses 510 and 511, the hot mirror 104, and the objective lens 303 through a path opposite to that during light application. Further, the light is reflected by the dichroic mirror 304 and the hot mirror 506, passes through the optical axis O2, is transmitted through the parallel flat glass 505, then reflected by the perforated mirror 504, and transmitted through a lens 507. Thereafter, the light forms an image in a ring shape (ring image) via a ring lens 508 by a two-dimensional image sensor (CCD) 509. The light source 501 employs infrared light having a wavelength longer than that of the alignment light (light source 101) and the observation light (light sources 301a and 302a). In this Example, an SLD (super luminescent diode) having a wavelength of 870 nm is employed. However, the light source is not limited to this, and an LED employed in the light source 101 or the like, or a laser diode (LD) may be employed.

Here, the parallel flat glass 505 is arranged at a position conjugate with the pupil of the eye E to be examined. When incident on the parallel flat glass 505 arranged obliquely with respect to the optical axis O2, the reflected light (light from the light source 501) is refracted and deviated by a predetermined distance (for example, ΔH) with respect to the optical axis O2. As described above, the parallel flat glass 505 rotates around the optical axis O2, and thus the reflected light transmitted through the parallel flat glass 505 rotates with a radius ΔH at a position of the parallel flat glass 505. Since the parallel flat glass 505 is arranged at a position conjugate with the position of the pupil of the eye E to be examined, the reflected light is applied to the retina of the eye to be examined while rotating with a predetermined (constant) radius (for example, Δh) at the position of the pupil of the eye E to be examined. Therefore, the reflected light forms, on the retina of the eye E to be examined, an image in a circular shape having a size and a shape corresponding to the eye refractive power of the eye E to be examined. Since the CCD 509 is arranged at a position conjugate with the retina of the eye E to be examined, the eye refractive power of the eye to be examined can be obtained by analyzing the ring image acquired by the CCD 509.

### Photographing optical system 300 at the time of photographing using light source 300a

In the ophthalmic device 1 according to this embodiment, in addition to the above measurements, it is possible to perform observation of a tear meniscus. The light source 300a is attached to the tip of the eyepiece part (the eye E side to be examined), and in this embodiment, the light source 300a is configured to project a ring-shaped pattern with the optical axis as the center. In this embodiment, the ring-shaped pattern has a plurality of rings which are different in diameter. When the distance between the eye to be examined and the light source 300a is constant, the range of the region that can be measured by the ring increases as the diameter of the ring increases. Of course, the light source 300a may be configured in various manners, and may have one light source that outputs light from a plurality of rings of a ring-shaped pattern or have a plurality of light sources corresponding to the plurality of rings, respectively.

For this reason, when the light source 300a is turned on, light is applied from multiple directions to the eye to be examined and the tear meniscus. In this embodiment, the light source 300a outputs white light. The white light has only to have a spectral distribution such that the eye to be examined irradiated with the white light is visually recognized in full color, and the color temperature and the light intensity for each wavelength are not limited. Note that the light source 300a only needs to be able to illuminate the region to be measured so that the region to be measured is photographed by the two-dimensional image sensor 306. Therefore, the wavelength of the light source 300a is not limited. For example, the light source 300a may be a light source that outputs infrared rays.

In this embodiment, when the photographing optical system 300 is used at the time of measuring the tear meniscus, the eyepiece part including the flat glasses 510 and 511 is set in front of the eye E to be examined as shown in FIG. 2. In this state, when the tear meniscus is irradiated with light from the light source 300a, an anterior ocular image of the eye to be examined is acquired by the objective lens 303, the imaging lens 305, and the two-dimensional image sensor 306.

In this embodiment, the target to be illuminated with the light source 300a and measured based on the taken image is a specific region in the eye E to be examined. Specifically, a tear meniscus is a target to be measured. The tear meniscus is tear fluid accumulated between the lower eyelid (or upper eyelid) edge and the eyeball of the eye to be examined. Therefore, for example, at the time of measuring the tear meniscus on the lower eyelid, it is not necessary to photograph the entire eye to be examined. For this reason, when the eye to be examined is photographed by the photographing optical system 300 in this embodiment, the entire eye to be examined may not be photographed by the two-dimensional image sensor 306. Specifically, in the photographing optical system 300 according to this embodiment, the two-dimensional image sensor 306 and optical component each having a size sufficient for observing a specific region where the intraocular pressure, eye refractive power, and tear meniscus are to be measured are selected. On the other hand, the entire eyeball of a person having a statistically large eye to be examined (having a long distance between the upper eyelid and the lower eyelid when the eyes are open) is allowed not to come into the visual field of the two-dimensional image sensor 306, and the size of the two-dimensional image sensor 306, the optical component, and the optical path are configured not to be excessively large.

Therefore, in this embodiment, it is guaranteed that the periphery of the corneal apex of the eye to be examined is photographed with the photographing optical system 300 in a state in which alignment has been completed, but it is not guaranteed that the tear meniscus portion can be photographed, and that the tear meniscus can be measured from the taken image. Therefore, in this embodiment, after the alignment is automatically performed, the photographing optical system 300 is further moved so that the tear meniscus can be reliably photographed.

### (2) Movement control processing:

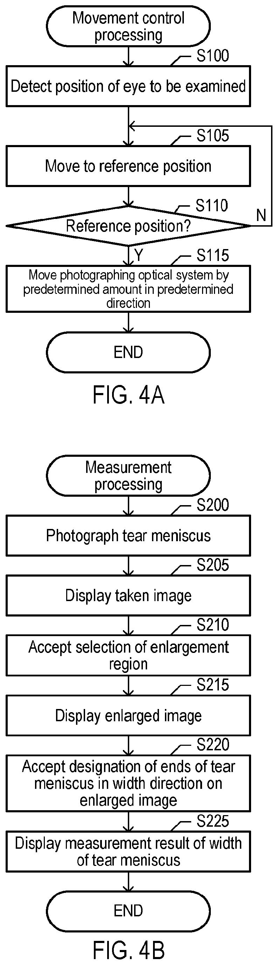

Hereinafter, movement control processing for moving the photographing optical system 300 to take an image for measuring the tear meniscus will be described. FIG. 4A is a flowchart showing movement control processing. The movement control processing is realized by the control unit 600 executing a control program (not shown). When the control program is executed, the control unit 600 functions as a reference movement control unit 600a, a displacement movement control unit 600b, and a measurement unit 600c. The reference movement control unit 600a is a program module that causes the control unit 600 to execute a function of automatically moving the photographing optical system to a reference position for photographing the reference region in the eye to be examined. The displacement movement control unit 600b is a program module that causes the control unit 600 to execute a function of automatically moving the photographing optical system to a displacement position different from the reference position.

When the subject sets his/her head to a predetermined position of the head part 602, and the examiner instructs the start of observation in the measurement of the tear meniscus, the control unit 600 starts the movement control processing.

When the movement control processing is started, the control unit 600 starts an alignment motion by the function of the reference movement control unit 600a.

In the alignment motion, the control unit 600 first detects the position of the eye to be examined by the function of the reference movement control unit 600a (step S100). Specifically, the control unit 600 turns on the light source 101. As a result, the infrared light from the light source 101 is applied to the cornea of the eye E to be examined through the hot mirror 102, the objective lens 103, and the hot mirror 104. The infrared light is reflected by the cornea, and received by the profile sensor 107 via the lens 105 and received by the profile sensor 108 via the lens 106. The control unit 600 detects the position of the eye E to be examined based on the reflected light received by the profile sensors 107 and 108.

Next, the control unit 600 moves the head part 602 including the photographing optical system 300 to a reference position (step S105). That is, the control unit 600 instructs the XYZ drive control unit 630 to move the head part 602. At this time, the control unit 600 instructs the XYZ drive control unit 630 so that the head part 602 moves by a predetermined amount toward the reference position determined in advance as the position where the eye E to be examined can be photographed by the photographing optical system 300. Specifically, when the head part 602 exists at the reference position, the positions where the reflected light from the cornea is received on the profile sensors 107 and 108 are determined in advance. Therefore, the control unit 600 moves the head part 602 by a predetermined amount so that the reflected light from the cornea moves toward the position determined in advance.

Next, the control unit 600 determines whether the head part 602 has reached the reference position (step S110). That is, the control unit 600 determines that the head part 602 has reached the reference position when the positions of the reflected light received by the profile sensors 107 and 108 are the positions determined in advance. When it is not determined in step S110 that the head part 602 has reached the reference position, the control unit 600 repeats the processing in step S105 and the subsequent steps.

On the other hand, if it is determined in step S110 that the head part 602 has reached the reference position, the photographing optical system 300 is brought into a state in which it exists at the reference position where the cornea, which is the reference region of the eye to be examined., can be photographed by the two-dimensional image sensor 306. In this state, it is guaranteed that the cornea can be photographed by the two-dimensional image sensor 306, but it is not guaranteed that the tear meniscus on the lower eyelid can be measured. For example, the lower eyelid of a person with large eyes is outside the visual field of the two-dimensional image sensor 306, or is photographed by a lens portion near edge with large distortion.

Therefore, the control unit 600 moves the head part 602 including the photographing optical system 300 by a predetermined amount in a predetermined direction by the function of the displacement movement control unit 600b (step S115). The predetermined direction and the predetermined amount only need to be determined in advance. For example, in this example in which the tear meniscus on the lower eyelid is photographed, the control unit 600 moves the photographing optical system 300 vertically from the position for photographing the corneal apex. Further, in this example in which the tear meniscus on the lower eyelid is photographed, the amount of displacement of the photographing optical system 300 from the reference position for photographing the corneal apex to the displacement position for photographing the position of the lower eyelid is specified in advance as the predetermined amount.

Therefore, the control unit 600 instructs the XYZ drive control unit 630 to move the head part 602 by the predetermined amount in a downward direction which is the predetermined direction. As a result, the photographing optical system 300 is moved to the displacement position where the tear meniscus on the lower eyelid is to be photographed by the two-dimensional image sensor 306, regardless of whether the person has large eyes or small eyes. With the above control processing, the photographing optical system 300 is moved to the displacement position where the tear meniscus is to be photographed.

### (3) Measurement processing:

This embodiment involves moving the photographing optical system 300 as described above, then photographing an image of the tear meniscus and measuring the width of the tear meniscus. That is, when the movement of the photographing optical system 300 is completed, the control unit 600 executes a control program (not shown). As a result, the control unit 600 functions as a measurement unit 600c that measures the tear meniscus based on the image of reflected light from the tear meniscus appearing in the image taken by illuminating the tear meniscus of the eye to be examined by the photographing optical system 300 existing at the displacement position.

FIG. 4B is a flowchart showing measurement processing executed by the control unit 600 using the function of the measurement unit 600c. When the measurement processing is started, the control unit 600 photographs the tear meniscus by the function of the measurement unit 600c (step S200). That is, the control unit 600 controls a lens driving unit in the photographing optical system 300 by the function of the measurement unit 600c, and moves at least one of the objective lens 303 and the imaging lens 305 in the optical axis direction to bring the tear meniscus into a focused state. The control unit 600 controls the two-dimensional image sensor 306 for photographing. As a result, an image of the tear meniscus is taken. Of course, the processing for realizing the focused state may include various types of processing. It is also possible to perform photographing in the focused state, or to take a plurality of image while changing the position of the lens and select an image in the focused state. When photographing is performed, the control unit 600 records the image output from the two-dimensional image sensor 306 in the memory 670.

Next, the control unit 600 displays the taken image by the function of the measurement unit 600c (step S205). That is, the control unit 600 controls the display unit 650 to display the taken image acquired in step S200. In this embodiment, the image of the tear meniscus can be displayed on the display unit 650 in a predetermined size, and the portion selected by the examiner on the image can be enlarged and displayed.

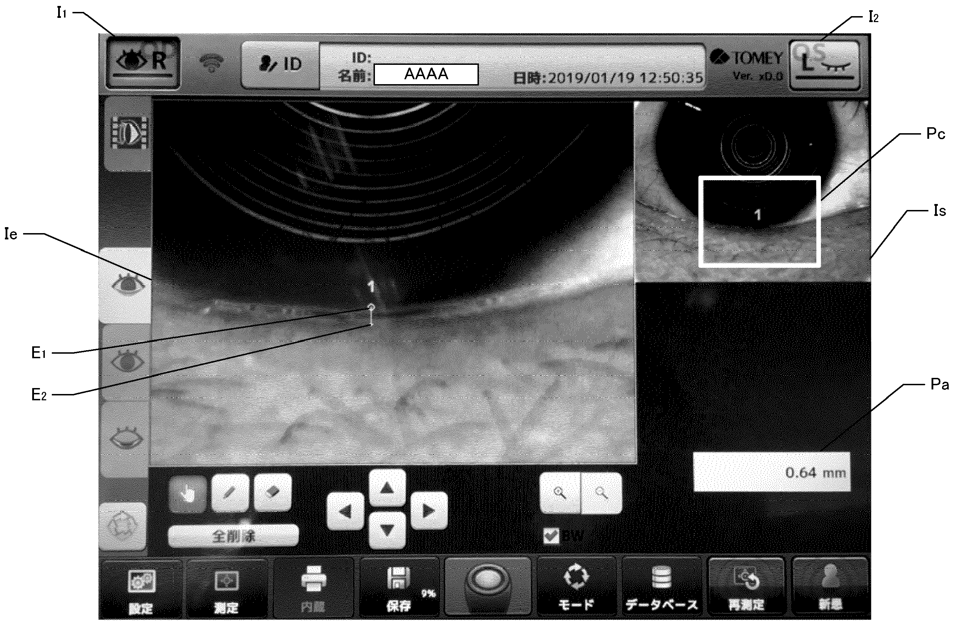

In order to realize such a configuration, in step S205, the control unit 600 causes the display unit 650 to display the image before enlargement. FIG. 5 is a diagram illustrating an example of a screen for displaying the taken image. In FIG. 5, a configuration is adopted in which images taken for the right eye and the left eye, respectively, of a subject can be selected and displayed. In FIG. 5, an icon I₁ indicated as R represents the right eye, and an icon I₂ indicated as L represents the left eye, and the image identified by the icon in which the eye is open is displayed, and the image identified by the icon in which the eye is closed is not displayed. Therefore, the example shown in FIG. 5 is the image of the right eye.

Furthermore, in the example shown in FIG. 5, an image Is before enlargement is displayed on the upper right side. Therefore, at the stage of step S205, the image Is is displayed in the screen shown in FIG. 5, but an image Ie is not displayed. In this embodiment, the control unit 600 may cause the display unit 650 to display the image of the tear meniscus as a monochrome image, or cause the display unit 650 to display it as a color image. Alternatively, it may be selectable whether the image of the tear meniscus is displayed as a monochrome image or a color image.

Next, the control unit 600 accepts selection of an enlargement region by the function of the measurement unit 600c (step S210). That is, the control unit 600 accepts an examiner's operation of the joystick 640 and/or the touch panel 660, and accepts selection of the enlargement region that is a region to be enlarged within the image Is before enlargement. In this embodiment, the target to be measured is the tear meniscus on the lower eyelid. Therefore, the examiner operates the joystick 640 and/or the touch panel 660 to designate a portion including the tear meniscus on the lower eyelid. In FIG. 5, the designated region is shown as a rectangle Pc.

Next, the control unit 600 displays an enlarged image using the function of the measurement unit 600c (step S215). That is, the control unit 600 controls the display unit 650 to enlarge and display the portion accepted in step S210. Specifically, the control unit 600 refers to the memory 670 and applies the enlargement processing to the image in the enlargement region accepted in step S210. The enlargement processing may include various types of interpolation processing. Note that, when the resolution of the taken image is sufficiently high and the display of the image in step S205 is display of the taken image in a size-reduced manner, the image may be displayed without performing any interpolation processing or the like during the display in step S215. Even in this case, it can be said that the display in step S205 is display before enlargement, and that the image is displayed in an enlarged manner in step S215. In the example shown in FIG. 5, an enlarged image Ie is displayed on the left side of the screen.

Next, the control unit 600 accepts designation of ends of the tear meniscus in the width direction on the enlarged image by the function of the measurement unit 600c (S220). That is, in this embodiment, the examiner can designate the ends of the tear meniscus in the width direction on the enlarged and displayed image. For this purpose, the control unit 600 accepts an examiner's operation of the joystick 640 and/or the touch panel 660, the examiner visually recognizes the ends of the tear meniscus in the width direction in the enlarged image Ie, and designates two ends in the width direction. In FIG. 5, the two designated ends are indicated as E₁ and E₂. As described above, according to the configuration of designating the ends of the tear meniscus in the enlarged view, the ends of a very small tear meniscus can be easily designated and can be designated accurately.

Next, the control unit 600 displays the measurement result of the width of the tear meniscus by the function of the measurement unit 600c (step S225). That is, the control unit 600 acquires the distance between the ends accepted in step S220 as the width of the tear meniscus. The width may be acquired by various techniques. For example, a configuration in which the correspondence between one pixel in the enlarged image and the distance in the actual space is identified in advance may be employed.

When the width of the tear meniscus is acquired, the control unit 600 controls the display unit 650 to display the width of the tear meniscus, by the function of the measurement unit 600c. In FIG. 5, a display part Pa that displays the measured width of the tear meniscus is provided, and the control unit 600 causes the display part Pa to display the width of the tear meniscus in units of length. According to the above configuration, even for a subject who has eyes having any size, it is possible to photograph the tear meniscus and easily measure the width of the tear meniscus.

### (4) Other Embodiments:

The above embodiment is an example for carrying out the present invention, and various other embodiments can be adopted as long as a specific region in the eye to be examined can be photographed by displacing the automatically aligned photographing optical system. For example, the ophthalmic device is not limited to the configuration including the head part and the main body part as described above, and may be an ophthalmic device additionally including other various elements. In addition, the tear meniscus measurement screen as shown in FIG. 5 is an example, and the width of the tear meniscus may be measured and displayed at a plurality of locations, or various statistical values may be displayed.

The photographing optical system only needs to be able to illuminate and photograph the eye to be examined. Specifically, the photographing optical system includes a light source that illuminates the eye to be examined, a sensor that images reflected light reflected by the eye to be examined, and an optical component between the light source and the sensor. Of course, the photographing optical system may employ various configurations other than the embodiment described above. For example, in the embodiment described above, a ring-shaped illumination is used as the light source, but illuminations of other shapes may be used. That is, it is only necessary that illumination be performed so that the light reflected from the target to be measured reaches the sensor. Of course, the photographing optical system may or need not share components with the optical systems for performing various measurements (in the embodiment described above, the intraocular pressure and the eye refractive power).

The reference movement control unit only needs to be able to automatically move the photographing optical system to the reference position for photographing the reference region in the eye to be examined. In other words, even if an attempt is made to measure the target to be measured in a state where the photographing optical system exists at the displacement position, the photographing optical system cannot be positioned at the displacement position for photographing the target if the position of the eye to be examined is unknown. Therefore, if the ophthalmic device has an automatic alignment function of automatically moving the photographing optical system to the reference position, the displacement position can be easily identified with reference to the reference position.

The reference region in the eye to be examined may be any region that serves as a reference for determining the position of the photographing optical system, and various configurations other than the configuration in which the reference region is the corneal apex as in the embodiment described above may be adopted. For example, various regions such as a medial canthus of eye, a lateral canthus of eye, and an end of an eyebrow may serve as the reference region. The reference position may be any position for photographing the reference region, and it is only necessary that the reference region can be photographed by a sensor provided by the photographing optical system. Accordingly, the state of the lens of the photographing optical system is arbitrary, and may be either a focused state or a non-focused state.

As the configuration for automatically moving the photographing optical system to the reference position, various techniques other than the configuration of the embodiment described above can be employed. For example, it is possible to employ a configuration of analyzing an image photographed by the photographing optical system and moving the photographing optical system to a position where the reference region is photographed by a specific region of the image sensor. The moving direction of the photographing optical system may be an arbitrary direction in the three-dimensional space, or may be any two-dimensional or one-dimensional direction.

The displacement movement control unit only needs to be able to move the photographing optical system to the displacement position different from the reference position. That is, in a state where the photographing optical system is present at the reference position, the photographing optical system and the reference region in the eye to be examined have a predetermined relationship. In the case where a specific region in the eye to be examined is to be measured, the specific regions in the eyes to be examined of all (or most) subjects are in nearly the same position, and it is possible to identify in advance how much the photographing optical system should be moved from the reference position. Therefore, when the position displacement between the displacement position where the specific regions in the eyes to be examined of all (or most) subjects can be photographed and the reference position is clarified, the movement corresponding to the position displacement is performed, so that the photographing optical system can be automatically moved to the displacement position.

The predetermined direction when the displacement movement control unit moves the photographing optical system may be any direction for moving the photographing optical system existing at the reference position to the displacement position, and may be determined in advance. If there is a plurality of regions to be measured, the predetermined direction may be determined for each target to be measured. The predetermined amount when the displacement movement control unit moves the photographing optical system may be any movement amount when the photographing optical system existing at the reference position is moved to the displacement position, and may be determined in advance. If there is a plurality of regions to be measured, the predetermined amount may be determined for each target to be measured.

The measurement unit only needs to be able to measure the tear meniscus based on the image of reflected light from the tear meniscus appearing in the image taken by illuminating the tear meniscus of the eye to be examined by the photographing optical system existing at the displacement position. That is, various configurations other than the configuration of the embodiment described above in which the width of the tear meniscus is measured based on the distance between the ends of the tear meniscus designated by the examiner can be employed. For example, a configuration may be employed in which the image processing is performed based on the photographed image, the image of the tear meniscus is identified based on the characteristics of the image of the tear meniscus, and the width of the tear meniscus is measured based on the image. The image processing for specifying the image of the tear meniscus may include various types of processing, and may be, for example, pattern matching or processing by artificial intelligence using machine learning or the like.

Furthermore, a technique of enabling photographing of a specific region in the eye to be examined by displacing the automatically aligned photographing optical system is also applicable as a method invention. The ophthalmic device and method as described above can be realized as a single apparatus or as a part of an apparatus having a plurality of functions, and includes various modes.

### Reference Signs List:

FIGS. 1, 2
EYEPIECE PART
FIG. 3
   602 HEAD PART
   100 ALIGNMENT OPTICAL SYSTEM
   300 PHOTOGRAPHING OPTICAL SYSTEM
   400 FIXATION OPTICAL SYSTEM
   200 DISPLACEMENT/DEFORMATION DETECTION LIGHT RECEIVING OPTICAL SYSTEM
   500 EYE REFRACTIVE POWER OPTICAL SYSTEM
   600 CONTROL UNIT
   600a REFERENCE MOVEMENT CONTROL UNIT
   600b DISPLACEMENT MOVEMENT CONTROL UNIT
   600c MEASUREMENT UNIT
   601 MAIN BODY PART
   630 XYZ DRIVE CONTROL UNIT
   640 JOYSTICK
   650 DISPLAY UNIT
   660 TOUCH PANEL
   670 MEMORY
   680 FIXATION TARGET CONTROL UNIT
FIG. 4A
   MOVEMENT CONTROL PROCESSING
   S100 DETECT POSITION OF EYE TO BE EXAMINED
   S105 MOVE TO REFERENCE POSITION
   S110 REFERENCE POSITION?
   S115 MOVE PHOTOGRAPHING OPTICAL SYSTEM BY PREDETERMINED AMOUNT IN PREDETERMINED DIRECTION
FIG. 4B
   MEASUREMENT PROCESSING
   S200 PHOTOGRAPH TEAR MENISCUS
   S205 DISPLAY TAKEN IMAGE
   S210 ACCEPT SELECTION OF ENLARGEMENT REGION
   S215 DISPLAY ENLARGED IMAGE
   S220 ACCEPT DESIGNATION OF ENDS OF TEAR MENISCUS IN WIDTH DIRECTION ON ENLARGED IMAGE
   S225 DISPLAY MEASUREMENT RESULT OF WIDTH OF TEAR MENISCUS

## Claims

1. An ophthalmic device comprising:
a photographing optical system that illuminates and photographs an eye to be examined:
a reference movement control unit that automatically moves the photographing optical system to a reference position for photographing a reference region in the eye to be examined; and
a displacement movement control unit that automatically moves the photographing optical system to a displacement position different from the reference position.

2. The ophthalmic device according to claim 1, wherein the displacement movement control unit moves the photographing optical system by a predetermined amount in a predetermined direction.

3. The ophthalmic device according to claim 1 or 2, further comprising a measurement unit that measures a tear meniscus based on an image of reflected light from the tear meniscus included in an image taken by illuminating the tear meniscus of the eye to be examined by the photographing optical system existing at the displacement position.

4. The ophthalmic device according to claim 3, wherein the measurement unit:
causes a display unit to display the image; and
measures the width of the tear meniscus based on the distance of ends of the tear meniscus in the width direction designated on the image by an examiner.

5. The ophthalmic device according to claim 4, wherein
the measurement unit enlarges the image and causes the display unit to display the enlarged image, and
the examiner designates the ends of the tear meniscus in the width direction on the enlarged and displayed image.

6. The ophthalmic device according to claim 5, wherein the measurement unit:
causes the display unit to display the image before enlargement; and
when the examiner designates a portion to be enlarged on the image before enlargement, enlarges the portion to be enlarged in the image and causes the display unit to display the enlarged image.
